# EUROPEAN PATENT APPLICATION

(11) **EP 2 028 491 A1**
(43) Date of publication of application: **25.02.2009**
(21) Application number: 07114771.4
(22) Date of filing: 22.08.2007
(51) Int. Cl.: G01N 33/543, G01R 33/09

(54) **Method of directing magnetic or magnetisable objects to prepare bio-sensor device**

(71) Applicant: Koninklijke Philips Electronics N.V., 5621 BA Eindhoven (NL)
(72) Inventor: The designation of the inventor has not yet been filed
(74) Representative: van Wermeskerken, Stephanie Christine

(57) **Abstract**

A method of preparing a biosensor device having magnetic or magnetisable particles (60) for binding to a target being detected, and having a magnetic field sensor (40) for detecting the magnetic or magnetisable particles (60) after binding, by depositing (410) the magnetic or magnetisable particles (60) on the device, and using a magnetic field to direct (420) the magnetic or magnetisable particles (60) to a free area on the device away from the magnetic field sensor (40), to be available for later use in binding and detecting. This can improve precision of location of the magnetic or magnetisable particles (60) or reduce manufacturing time. The device can be an integrated micro-fluidic device. It can have a built in magnetic field generation means comprising a wire (260, 270) integrated on an integrated circuit (10, 30) of the device.

## Description

### FIELD OF THE INVENTION

The invention relates to methods of preparing bio-sensor devices having magnetic or magnetisable objects, e.g. magnetic or magnetisable particles, for binding to a target being detected, and having a magnetic field sensor for detecting the magnetic or magnetisable objects, e.g. magnetic or magnetisable particles, after binding, and to corresponding devices.

### BACKGROUND OF THE INVENTION

Medical diagnostics, both in the central laboratory and at the bedside, is driven towards more integration and automation. The reason for this is that tests need to be easy to perform, in a reliable and cost effective way, with minimum human intervention. At the same time there is an ever- increasing need for higher sensitivity and specificity of detection.

Biosensor devices such as magnetic biochips, have been proposed as a new means to sensitively detect low concentrations of target molecules in body fluids for diagnostics. Such magnetic biochips have promising properties for bio-molecular diagnostics, in terms of sensitivity, specificity, integration, ease of use and costs. For example, sensitive magneto-resistive magnetic field sensors, such as GMR magnetic field sensors, can be combined with suitable biochemistry to selectively attach magnetic beads, resulting in a miniaturized biosensor that is suitable for detection in an array format. The sensitivity and specificity of rapid tests is usually provided by dedicated capture of probe molecules, e.g. a high-affinity antibody-antigen combination. In such an immunoassay, the target molecules become sandwiched between antibodies on a solid support and a label that is detected by the sensor. Conventionally this label is a fluorophore, and a plate reader is used for detection. In the most sensitive assays, the test is performed on magnetic bead carriers that can be actuated so the reaction rate is no longer limited by diffusion and the test is speeded up. Magnetic detection naturally combines actuation and detection by using the magnetic beads as both label and carrier. Besides this natural integration, magnetic labelling has several other advantages: body liquids do show autofluorescence, but are by nature hardly magnetic, which helps to improve the detection limit. Magnetic detection of magnetic particles requires no expensive optics, yet is fast and sensitive, and furthermore, it is well suited for miniaturized diagnostic sensing, due to the direct availability of electronic signals and the small size of the required instrumentation. The aim of a biosensor is to detect and quantify the presence of a biological molecule in a sample, usually a solution. Desired attributes are high sensitivity, high specificity, and high speed. Furthermore, the biosensor is preferably of low cost and it should be reliable and easy to use. For many decades magnetic particles have been used in biology for separation, extraction and purification of biological materials. In recent years, biosensors based on the use of magnetic particles for actuation as well as detection have been developed. In studies, magnetic particles are detected by optical methods, electric means, coils or magneto- resistive sensors. Actuation of the particles is used for stringency, to concentrate the particles near the detection surface or to enhance particle-to-particle binding.

An example of a high sensitivity and high speed sandwich assay is mentioned in patent application WO2006079998 by the present applicants, using the following steps:
Disperse: Mix beads into the fluid sample.

Catch: Let targets bind to beads.

Attract: Bring beads toward the binding surface of the sensor device.

Bind: Let beads form biological bonds to the binding region or binding sites of the sensor.

The binding surface of the sensor has a portion which is chemically and/or biochemically prepared to enable binding of particles, in particular via selective biochemical bonds. In the case of a sandwich format, the biochemical bond involves at least the following elements: an area on the sensor binding surface, a first binding entity, a target, a second binding entity, and the bead.

Stringency (i.e. selective removal): Remove unbound and weakly bound beads from the binding surface of the sensor device, either magnetically or non-magnetically. Determine relative binding forces. The catching step can be made very fast by providing a high surface-to- volume ratio, i.e. by dispersing many small particles in the solution. However, this is only useful when the other steps can also be made very rapid. Thermal diffusion, however, gives only slow transport of sub-micrometer beads towards the binding surface. The transport can be enhanced by applying magnetic field gradients, e.g. with a permanent magnet [as in Perrin, J.Immun.Meth 224, 77 (1999) for example].

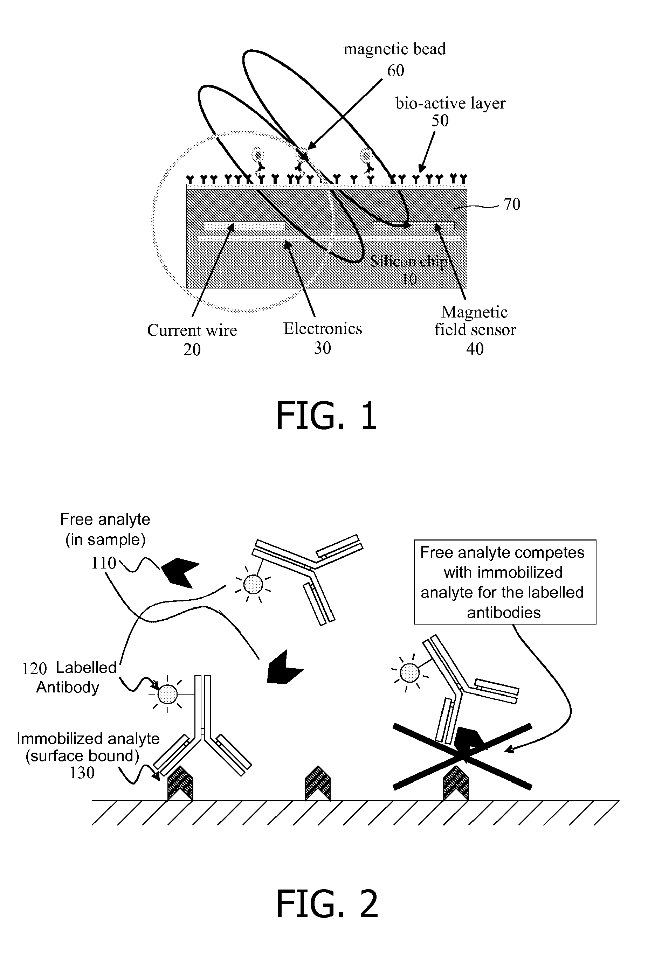

Furthermore, a biosensor comprising an array of sensors, e.g. 100 sensors, based on the magnetic detection of magnetic beads, e.g. superparamagnetic beads, may be used to simultaneously measure the concentration of a large number of different biological molecules (e.g. proteins, DNA) in a solution (e.g. blood). This may be achieved by attaching magnetic beads to target molecule, magnetising these beads with an applied magnetic field and using a Giant Magneto Resistance (GMR) sensor to detect the stray field of the magnetised beads, which stray field is dependent on the concentration. Biosensor devices can be used to detect analytes in biological fluids such as blood, plasma, urine, saliva, sweat, etc. Examples of biosensor can comprise a sensing element present in a substrate, arranged to carry out a sandwich assay, as shown schematically in figure 1 as will be described in more detail below.

For the detection of small molecules, such a sandwich assay is not possible because the analyte does not have two binding sites for antibodies. As an alternative a competitive, inhibitive or displacement assay can be used. Its operation is described below with reference to figure 2.

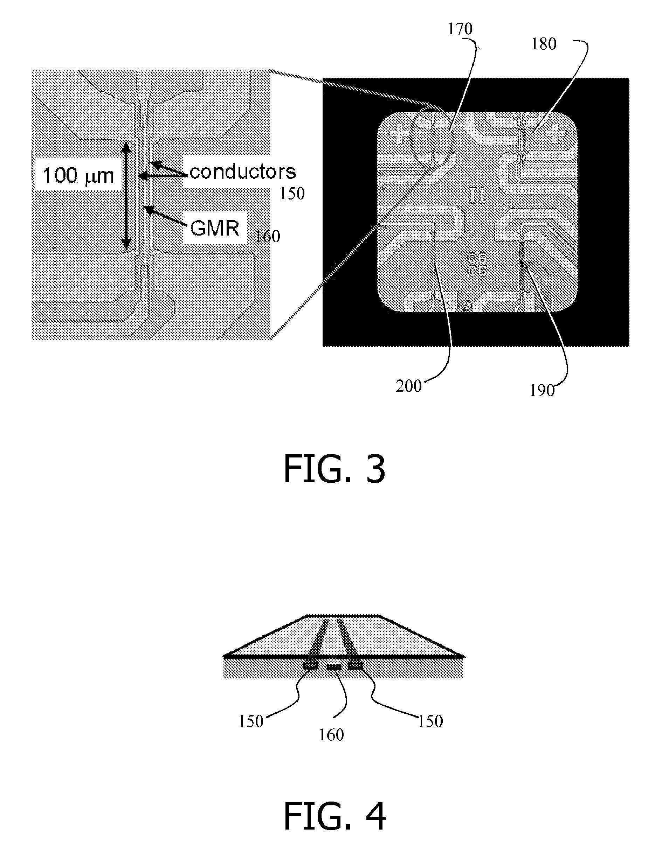

For many diagnostics applications it is preferable to detect multiple analytes with a single test. This means that different antibodies (in case of a sandwich assay) or different analytes (in case of a competitive or inhibit assay) must be immobilized on a single chip surface. Such a chip may contain many different sensors. An example containing four sensors is shown in the plan view photograph of figure 3 and the perspective view of figure 4, described in more detail below. One way to do the spotting of multiple areas with different chemicals is by printing with a bio-inkjet printer. Alternatives are pin-transfer or acoustic liquid dispensing.

### SUMMARY OF THE INVENTION

An object of embodiments of the invention is to provide good apparatus or methods.

According to a first aspect, the invention provides:

A method of preparing a biosensor device having magnetic or magnetisable objects, e.g. magnetic or magnetizable particles, for binding to a target being detected, and having a magnetic field sensor for detecting the magnetic or magnetisable objects, e.g. magnetic or magnetizable particles after binding, the method having the step of depositing the magnetic or magnetisable objects, e.g. magnetic or magnetizable particles on the device, and using a magnetic field to direct the magnetic or magnetisable objects, e.g. magnetic or magnetizable particles to a free area on the device away from the magnetic field sensor, to be available for later use in binding and detecting.

Compared to known methods of applying by bio-inkjet printing for example, the use of a magnetic field can improve precision of location of the magnetic or magnetisable objects, e.g. magnetic or magnetizable particles or reduce manufacturing time or reduce costs. Although it is known to direct the magnetic or magnetisable objects, e.g. magnetic or magnetizable particles by magnetic fields when the biosensor is in use, for directing the magnetic or magnetisable objects, e.g. magnetic or magnetizable particles towards the binding and detecting locations, there is no suggestion of using such magnetic fields to direct the particles during manufacture or preparation before any such binding or detection. Other advantages will be apparent for particular sets of embodiments or any embodiments when compared to different known methods.

Embodiments of the invention can have any additional features without departing from the scope of the claims. Some such additional features are set out in dependent claims and exemplified below. Other aspects of the invention include corresponding apparatus.

Any of the additional features can be combined together and combined with any of the aspects. Other advantages will be apparent to those skilled in the art, especially over other prior art. Numerous variations and modifications can be made without departing from the claims of the present invention. Therefore, it should be clearly understood that the form of the present invention is illustrative only and is not intended to limit the scope of the present invention.

### BRIEF DESCRIPTION OF THE DRAWINGS

How the present invention may be put into effect will now be described by way of example with reference to the appended drawings, in which:
Fig.. 1 shows an example of a sandwich assay,
Fig. 2 shows an example of operation of inhibitive or displacement assay,
Fig. 3 shows a layout of sensors,
Fig. 4 shows a perspective view of part of figure 3,
Fig. 5 shows steps of a method according to an embodiment,
Figs. 6 and 7 show embodiments with on-chip magnetic field generation,
Figs 8 to 11 show a sequence of four stages in preparing a bio-sensing device using an external coil or coils.

### DETAILED DESCRIPTION OF THE EMBODIMENTS

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

The term "comprising", used in the claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practised without these specific details. In other instances, well-known methods, structures and techniques have not been shown in detail in order not to obscure an understanding of this description.

In biosensing processes using a magnetic sensor device, magnetic or magnetisable objects, e.g. magnetic or magnetizable particles (which can be of any size, from molecules, to strings of molecules, to beads) are directly or indirectly attached to target molecules such as e.g. proteins, antibodies, nucleic acids (e.g. DNR, RNA), peptides, oligo-or polysaccharides or sugars, small molecules, hormones, drugs, metabolites, cells or cell fractions, tissue fractions. These molecules are to be detected in a fluid, which can be the original sample or can already have been processed before insertion into the biosensor (e.g. diluted, digested, degraded, biochemically modified, filtered, dissolved into a buffer). The original fluids can be for example, biological fluids, such as saliva, sputum, blood, blood plasma, interstitial fluid or urine, or other fluids such as drinking fluids, environmental fluids, or a fluid that results from sample pre-treatment. The fluid can for example comprise elements of solid sample material, e.g. from biopsies, stool, food, feed, environmental samples.

The present invention will be described by means of the magnetic or magnetisable objects being magnetic or magnetisable particles. Again, this is only for the ease of explanation and does not limit the invention in any way. The present invention also applies for a magnetic or magnetisable object being a magnetic rod, a string of magnetic particles, or a composite particle, e.g. a particle containing magnetic as well as optically-active material, or magnetic material inside a non-magnetic matrix.

The surface of the sensor device may be modified by a coating which is designed to attract certain molecules or by attaching molecules to it, which are suitable to bind the target molecules which are present in the fluid. Such molecules are know to the skilled person and include complementary DNA, antibodies, antisense RNA, etc. Such molecules may be attached to the surface by means of spacer or linker molecules. The surface of the sensor can also be provided with organisms (e.g. viruses or cells) or fractions of organisms (e.g. tissue fractions, cell fractions, membranes). The surface of biological binding can be in direct contact with the sensor chip, or there can also be a gap between the binding surface and the sensor chip. For example, the binding surface can be a material that is separated from the chip, e.g. a porous material. Such a material can be a lateral- flow or a flow-through material, e.g. consisting of microchannels in silicon, glass, plastic, etc. The binding surface can be parallel to the surface of the sensor chip. Alternatively, the binding surface can be at an angle, e.g. perpendicular to, the surface of the sensor chip.

Before the magnetic or magnetisable particles or the target molecules/magnetic or magnetic or magnetisable particles- combination can be bound to the surface of the sensor device, they have to be attracted towards that surface. It has to be noted that magnetic or magnetisable particles can be used in various types of assays, e.g. a binding or unbinding assay, sandwich assay, displacement assay, inhibition assay, or competition assay. In the following, an example using a binding assay, more specifically a sandwich assay will be discussed, but the described methods are not limited to this assay type.

### Figure 1, Sandwich assay

Figure 1 shows an example of a sandwich assay to which the embodiments can be applied as will be described. The present invention will be described by means of a substrate 10 being a silicon chip. It has to be understood that this is not intended to limit the invention in any way. In embodiments of the present invention, the term "substrate" may include any underlying material or materials that may be used, or upon which a device, a circuit or an epitaxial layer may be formed. In other alternative embodiments, this "substrate" may include a semiconductor substrate such as e.g. a doped silicon, a gallium arsenide (GaAs), a gallium arsenide phosphide (GaAsP), an indium phosphide (InP), a germanium (Ge), or a silicon germanium (SiGe) substrate. The "substrate" may include for example, an insulating layer such as a SiO₂ or an Si₃N₄ layer in addition to a semiconductor substrate portion. Thus, the term substrate also includes silicon-on-glass, silicon-on sapphire substrates. The term "substrate" is thus used to define generally the elements for layers that underlie a layer or portions of interest. Also, the "substrate" may be any other base on which a layer is formed, for example a glass or metal layer. The substrate may optionally be planarised. This may be done by for example depositing a planarisation layer of a photoresist, which may for example be an epoxy- or novolac-based polymer, onto the substrate.The substrate in the form of a silicon chip 10 has control circuitry in the form of integrated electronics 30 such as conventional analog or digital circuitry, covered by an insulating layer 70.

A magnetic field sensor 40 is also provided integrated on the substrate 10, and a current wire 20 for causing a magnetic field to interact with the magnetic or magnetisable particle 60. The insulating layer 70 is covered with a bio active layer 50 that is active in the total (immuno-) assay that is used for detection. An example of such a layer 50 is a surface functionalised with antibodies. These bind specifically to the target being sought. Then incubation is done with a secondary antibody labelled with a small magnetic or magnetisable particle 60. This secondary anti-body only binds when the target is present in the fluid. A washing step distinguishes between the secondary anti-bodies that have a specific binding and those that did not bind (or have non-specific binding). The magnetic or magnetisable particle 60 can be detected by measuring its in-plane stray field at the position of the magnetic field sensor 40.

In a biosensor assay, the 'attract' and 'bind' phases should be made as efficient and as fast as possible. In the 'attract' phase the beads are moved from their starting locations in free areas by means of a fluid, and concentrated from the bulk of the fluid to a zone near the sensor binding surface. The time needed to attract the magnetic or magnetisable particles 60 toward the binding surface should be as low as possible, lower than 30 minutes, preferably lower than 10 minutes, and more preferred lower than 1 minute.

In the 'bind' phase, the resulting bead ensemble is brought even closer to the binding surface in a way to optimise the occurrence of desired (bio)chemical binding to the capture or binding area on the sensor, i.e. the area where there is a high detection sensitivity by the sensors, e.g. magnetic sensors, and a high biological specificity of binding. It is desirable to have a good binding efficiency (the rate of specific biological binding when the bead is close to the binding surface) as well as the good contact time (the total time that individual beads are in contact with the binding surface).

The chip can also have micro fluidic elements integrated following established practice (not shown).

### Figure 2 operation of inhibitive or displacement assay

In Figure 2, an example of an inhibitive or displacement assay is shown. In the displacement assay, there is some analyte 130 immobilized on the sensor surface. There is also free analyte 110 in the biological fluid, which now competes with the immobilized analyte 130 or analyte-like on the surface for binding to the labelled antibody 120 (see right side of Fig. 2). This means that if no target is present in the fluid all antibodies will bind to the sensor surface and the signal is high, while when target molecules are present in the fluid less (or no) antibodies will bind to the sensor surface and the signal is low. A schematic drawing is shown in figure 2. The competitive assay is proposed for detecting drugs (morphine, opiates, amphetamines, etc.) in saliva.

### Figures 3 and 4 layout of sensors

Figure 3 shows in the right hand side a plan view of a substrate having four sensors 170, 180, 190, 200 and at the left side a zoomed plan view of one of the sensors 170. The sensor has a pair of current carrying conductors 150, and a magnetic field sensor in the form of an elongate GMR element 160. It has to be noted that this is not intended to limit the invention in any way. The present invention may be applied to sensors comprising any sensor element suitable for detecting the presence or determining the amount of magnetic or magnetizable objects, e.g. magnetic or magnetisable particles 60, on or near a sensor surface based on any property of the magnetic or magnetisable particles 60. For example, detection of the magnetic or magnetisable particles 60 may be done by means of magnetic methods (e.g. magnetoresistive sensor elements, hall sensors, coils), optical methods (e.g. imaging fluorescence, chemiluminescence, absorption, scattering, surface plasmon resonance, Raman, ...), sonic detection (e.g. surface acoustic wave, bulk acoustic wave, cantilever, quartz crystal, ...), electrical detection (e.g. conduction, impedance, amperometric, redox cycling), ....

Figure 4 shows a perspective view with a cut away to show how the conductors 150 and the GMR element 160 are buried. In Figure 3 various interconnect traces are shown for connecting the conductors 150 to other elements (not shown) or to contact points or a lead frame for off chip connections. The free areas can be located either in between the sensors 170, 180, 190, 200 or at the periphery of the substrate. Again micro-fluidic elements (not shown) can be incorporated above the substrate shown, to enable fluids to be transported to the region over each sensor 170, 180, 190, 200.

### Problems in depositing the magnetic or magnetisable particles 60

For fast assay applications (≤1 minute) it is necessary to start with the magnetic or magnetisable particles 60 located in a neighbourhood of the sensors 170, 180, 190, 200, such as at the free areas where there are no sensors 170, 180, 190, 200 or bioactive materials, for example between sensors 170, 180, 190, 200 or at the edges of the chip. Then in use, the magnetic or magnetisable particles 60 can be moved, typically picked up by a fluid flow, to reach the binding and detection locations. Manufacturing biosensor devices with such magnetic or magnetisable particles 60 in such free areas poses two different types of problems when conventional methods such as bio-inkjet printing, pin-transfer or acoustic liquid dispensing are used:
Precision of applying to the free areas a reproducible deposition of the magnetic or magnetisable particles 60 in solution.
Speed of deposition of the magnetic or magnetisable particles 60 in the context of mass production.
Embodiments of the invention involve directing the magnetic or magnetisable particles 60 during or after the deposition during manufacturing or preparation of the device. Several options of how to do this will be described.

### Figure 5

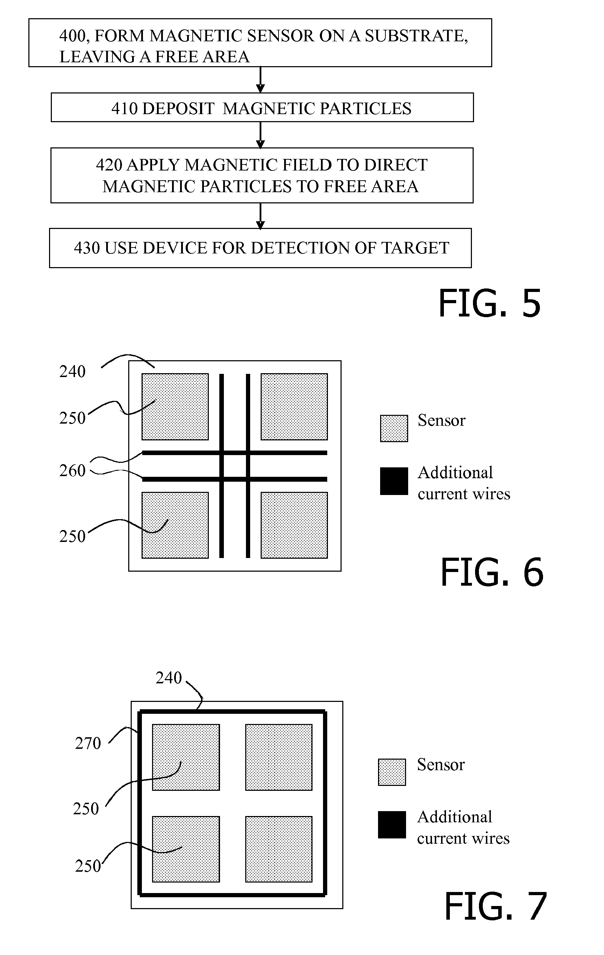

Figure 5 shows steps according to an embodiment. In a first step 400, the magnetic sensor is formed on a substrate 10, leaving a free area for the magnetic or magnetisable particles 60. In embodiments of the present invention, the term "substrate" may include any underlying material or materials that may be used, or upon which a device, a circuit or an epitaxial layer may be formed. In other alternative embodiments, this "substrate" may include a semiconductor substrate such as e.g. a doped silicon, a gallium arsenide (GaAs), a gallium arsenide phosphide (GaAsP), an indium phosphide (InP), a germanium (Ge), or a silicon germanium (SiGe) substrate. The "substrate" may include for example, an insulating layer such as a SiO₂ or an Si₃N₄ layer in addition to a semiconductor substrate portion. Thus, the term substrate also includes silicon-on-glass, silicon-on sapphire substrates. The term "substrate" is thus used to define generally the elements for layers that underlie a layer or portions of interest. Also, the "substrate" may be any other base on which a layer is formed, for example a glass or metal layer. The substrate may optionally be planarised. This may be done by for example depositing a planarisation layer of a photoresist, which may for example be an epoxy- or novolac-based polymer, onto the substrate.

If part of a process of manufacturing an integrated device, then there can be many other steps here, before or after forming the magnetic sensor. In a second step 410, the magnetic or magnetisable particles 60 are deposited on the device, typically on some part of the substrate 10. In a third step 420 a magnetic field is applied to direct the magnetic or magnetisable particles 60 to the free area, to be available for later use in binding and detecting. By moving the magnetic or magnetisable particles 60 away from the magnetic field sensor 40, the accuracy or reliability of the detection can be enhanced, since the detection relies on a difference between levels of magnetic field before and during the detection. This difference would be reduced if there were magnetic or magnetisable particles 60 too close to the magnetic field sensor 40 before the detection. This directing step can involve concentrating the magnetic or magnetisable particles 60 into a smaller area, or can involve moving them without concentrating. This third step can be carried out during deposition, to deflect magnetic or magnetisable particles 60 as they are deposited, or immediately after deposition if the magnetic or magnetisable particles 60 are able to move once deposited. Conceivably the magnetic or magnetisable particles 60 can be loosened later by a fluid for example, to enable the directing to take place much later than the deposition, as a configuration step carried out later, at any time before use of the device.

A fourth step 430 is the subsequent use of the device for the detection, by allowing the magnetic or magnetisable particles 60 to contact the target substance, to bind with the target, and for the presence or absence of the binding be detected by the magnetic field sensor 40. This use can take place in the field, in a laboratory, or anywhere.

As shown, this can address or ameliorate the problems related to bringing the deposited magnetic or magnetisable particles 60 (typically applied in a fluid solution or suspension) to the locations of the free areas. The speed of application of the magnetic or magnetisable particles 60 by deposition in mass production conditions can also be improved.

### Additional Features

An additional feature of some embodiments is the application of the magnetic or magnetisable particles 60 involving applying a fluid containing the magnetic or magnetisable particles 60 to a substrate 10 having the sensor 40 and the free area. In principle it is not essential to apply the magnetic or magnetisable particles 60 by using a fluid, but it is usually more practical.

Another such additional feature is the free area being adjacent to the sensor 40. In principle the free area can be further away, but being adjacent can help reduce transport time to the sensor 40. The free area can be between sensors 40 or at the edge of the substrate 10 for example, or conceivably above or below the sensor 40.

Another additional feature is the device being an integrated micro-fluidic device having fluid handling elements and control circuitry on the same substrate. The magnetic field sensor may be a magneto- resistive sensor element such as for example a GMR, TMR or AMR sensor element.

Magnetic fields with strong in-plane components may be applied by off-chip field-generating means, such as coils. Alternatively or as well, an on- chip field generation means can be provided, such by as wires integrated on the substrate, and/or wires integrated into packaging of the chip. On-chip wires have the notable advantage that any interference with the magnetic sensing element, e.g. a GMR, is well defined. However, it may be more expensive and complex to manufacture, and may add to the number of leads to the chip or to the chip package.

Examples will be described in which the magnetic field for moving the magnetic or magnetisable particles 60 is achieved using additional current wires and/or external magnetic fields are introduced. Furthermore the directing can be done following deposition of the magnetic or magnetisable particles 60 in a slow evaporating fluid additive. This can enable the directing to be carried out some time after the deposition. The current wires can be integrated in a chip, or integrated at another position in the packaging of the chip or cartridges.

### Figures 6, 7 embodiments with on-chip magnetic field generation:

Figures 6 and 7 show examples of arrangements of additional wires on the chip 10, arranged such that the magnetic or magnetisable particles 60 can be transported to their correct position or positions. The wires can be activated by driving an electric current, during or after deposition of the magnetic or magnetisable particles 60. The current can be controlled by on chip circuitry or by off-chip test circuitry as desired. Some examples of additional current wires in the chip design are shown in figures 6 and 7. In figure 6, a chip has a substrate 240 having four sensors 250, and wires 260 integrated on the substrate 240 for generating the field to direct the magnetic or magnetisable particles 60. The wires 260 are arranged in between the sensors 250, so that the magnetic or magnetisable particles 60 are attracted to the wires 260 and thus attracted to be located adjacent to the sensors 250, rather than over the centre of the sensors 250.

In figure 7, a similar arrangement is shown, but a single wire 270 is shown arranged around an outside edge of the group of sensors 250. There would be drive circuitry or a break in the circuit to connect to external drive circuitry (not shown).

In this way a solution with magnetic or magnetisable particles 60 can be applied with a (possibly automatic) pipette or syringe, whereby the accurate positioning is not important. The drop(s) with solution or suspension of magnetic or magnetisable particles 60 can be deposited somewhere on the chip near to the required final position: the additional current wires 260 can be driven to attract the magnetic or magnetisable particles 60 to the designed locations during the deposition process.

Another option is adding a slow evaporating solvent, whereby the actuation of the additional current wires 260 can be done as a separate step after deposition of the magnetic or magnetisable particles 60. In this way, the deposition process can go on while the actuation to direct the magnetic or magnetisable particles 60 could takes place later and optionally off-site. An example of slow-evaporating solvent is glycerol. 10 % v/v Concentration gives no negative effect on clustering of the magnetic or magnetisable particles 60.

In both ways the definitive location of the magnetic or magnetisable particles 60 can be more accurate. The solution with current wires 260 on the chip usually means additional bonding wires 260 to the chip. With off chip solutions this disadvantage can be overcome.

Note that the current wires 260 not necessarily need to be integrated with the chip. In an alternative embodiment the current wires 260 are integrated at another position in the chip packaging. As an example the current wires 260 can be integrated into a plastic package during an injection moulding process (similar to the moulding of lead-frames in chip packages). This could be a very cost-effective solution that offers a lot of freedom in the design of the current wires 260 and the resulting location of the magnetic or magnetisable particles 60.

On chip field generation by local current wires 260 are useful to make field gradients (unit T/m) with relatively small currents, e.g. smaller than 100 mA. It can be advantageous to use small currents in order to minimise the fields and particularly the in-plane fields applied to the magnetic field sensor 40. The application of high fields can change the sensitivity of the magnetic field sensor 40, and cause changes of its magnetic structure, e.g. domain wall changes, magnetic loops, or hysteresis. Furthermore it may be advantageous to generate these fields on the sensor chip so that the fields are well defined. This can help avoid a need for tight mechanical tolerances in the cartridge and read-out station.

In the absence of other field-generating means a current wire 260 with rectangular cross-section, may generate the highest fields and highest field gradients at its edges. This means that magnetic or magnetisable particles 60 near the edges of the current wire 260 will be more strongly attracted than magnetic or magnetisable particles 60 elsewhere.

The magnetic field generating means can comprise at least two current wires 260 wherein at least a first current wire 260 is positioned at a first side of the magnetic sensor element 160 and at least a second current wire 260 is positioned at a second side of the magnetic sensor element 160.

Many different ways of implementing the magnetic field generating means can be envisaged, some are described in patent application WO2006079998.

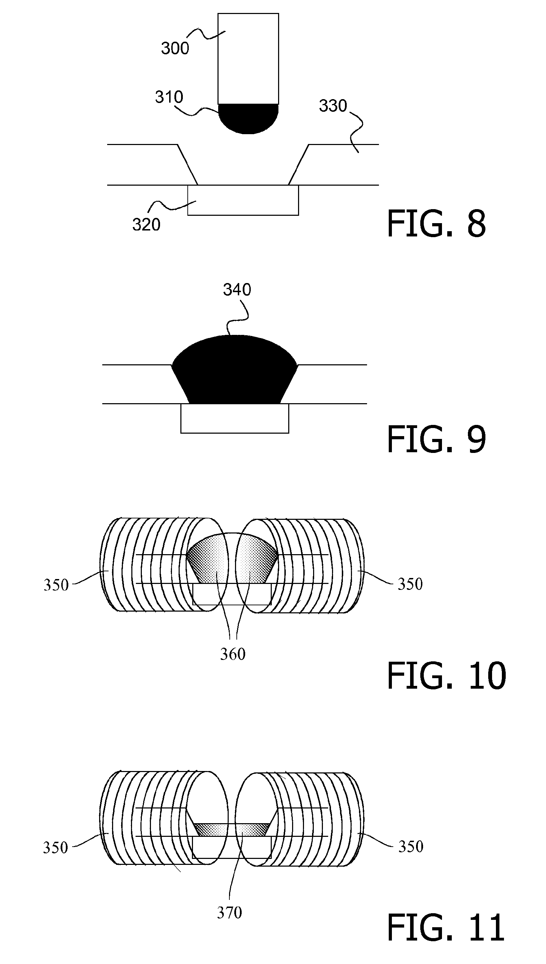

### Figures 8-11, off-chip magnetic field generation embodiments:

These figures show a sequence of four stages in preparing a bio-sensing device using an external coil or coils, starting with a deposition of a solution of magnetic or magnetisable particles 60, a view after deposition of the homogeneous magnetic or magnetisable particles solution, use of magnetic fields for transporting magnetic or magnetisable particles 60 to the edges, and in Figure 11, how to dry in the fluid until only magnetic or magnetisable particles 60 are on the edges. Figure 8 shows a deposition apparatus 300 such as an ink jet printer or droplet dispenser, for depositing a droplet 310 having the magnetic or magnetisable particles 60. The device being prepared has a chip 320 in a package 330 having an opening for receiving the droplet 310. The device package can be in the form of an MID (moulded interconnect device that contains the GMR chip). Figure 9 shows a view of the package after the droplet 310 has been deposited in the opening (indicated with reference number 340 in figure 9). Figure 10 shows how external coils 350 are placed over left and right sides of the package. When the coils 350 are driven, they create magnetic fields attracting the magnetic or magnetisable particles 60 in the droplet 310. As shown in figure 10, the result is that the magnetic or magnetisable particles 60 are attracted to both sides 360 of the chip, and thus away from the centre of the chip. If the sensors are arranged as in figure 3 or 6 or 7, then the magnetic or magnetisable particles 60 will be adjacent to the edges of the sensors rather than distributed over the centre of them. The magnetic or magnetisable particles 60 are pushed aside when the magnetic coils 350 are activated. If the solvent dries quickly, the magnetic or magnetisable particles 60 become fixed in place (indicated with reference number 370 in figure 11), and the coils 350 can then be deactivated.

The directing can be done during the deposition, or can be done after deposition by possible addition of a slow evaporating solvent. The disadvantage of this principle is that the absolute position of the magnetic or magnetisable particles 60 on the chip-in-MID may not be as accurate as in the case of current wires 260 on the chip or in the package. Another advantage is that the particle displacement is not very selective. All magnetic or magnetisable particles 60 are moved simultaneously. An advantage is that no additional electrical connections need to be made to the chip or to the package or cartridge during the application process. Note that the embodiments need not be limited to the configuration of external magnetic fields shown in the figures. Different magnetic field configurations are possible to guide the particles to the correct position on the device.

Bio-sensor devices can be implemented as a one part disposable device, or two-part system having a re-usable reader and a disposable unit in which the sample is entered. In this latter case, the magnetic field-generating coils can be part of the reader or of the disposable unit. In the disposable unit, the coil can be embedded in the outer material (generally plastic) or be integrated onto a chip that also performs the detection of the magnetic field of the magnetic or magnetisable particles 60. According to alternative embodiments, the sensor device may comprise at least one nonmagnetic sensor element, e.g. an optical sensor element.

### Concluding Remarks

Note that the embodiments are not limited to use with inkjet printing but are also applicable for other deposition methods (such as acoustic liquid dispensing or pin-transfer). Note that the scope is also not limited to the use in GMR magnetic biosensor cartridges, but can be used in any cartridge wherein magnetic or magnetisable particles 60 are used. Other variations and examples can be envisaged within the scope of the claims.

It is to be understood that although preferred embodiments, specific constructions and configurations, as well as materials, have been discussed herein for devices according to the present invention, various changes or modifications in form and detail may be made without departing from the scope of this invention as defined by the appended claims.

## Claims

1. A method of preparing a biosensor device having magnetic or magnetisable particles (60) for binding to a target being detected, and having a magnetic field sensor (40) for detecting the magnetic or magnetisable particles (60) after binding, the method having the step (410) of depositing the magnetic or magnetisable particles (60) on the device, and using a magnetic field to direct (420) the magnetic or magnetisable particles (60) to a free area on the device away from the magnetic field sensor (40), to be available for later use in binding and detecting.

2. The method of claim 1, and the deposition of the magnetic or magnetisable particles (60) involving depositing a fluid (310) containing the magnetic or magnetisable particles (60).

3. The method of claim 1 or 2, the free area being adjacent to the magnetic field sensor (40).

4. The method of any preceding claim, the device being an integrated micro-fluidic device having fluid handling elements and control circuitry (30) on the same substrate (10) as the magnetic field sensor (40).

5. The method of any preceding claim, the magnetic field sensor (40) comprising a magneto- resistive sensor element (160).

6. The method of any preceding claim, having the step of using an external magnetic field-generating means (350), to provide the magnetic field.

7. The method of any preceding claim, the device having a built in magnetic field generation means (260, 270), and the method having the step of using the built in magnetic field-generating means, to provide the magnetic field.

8. The method of claim 7, the built in magnetic field generation means comprising a wire (260, 270) integrated on an integrated circuit (30) of the device, and the method comprising driving a current in the wire (260, 270) to generate the magnetic field.

9. The method of claim 7, the built in magnetic field generation means comprising a wire (260, 270) integrated in packaging (330) of an integrated circuit (30) of the device, and the method comprising driving a current in the wire (260, 270) to generate the magnetic field.

10. The method of claim 2 or any preceding claim when depending on claim 2, and the directing being arranged to take place as a separate step after completion of the deposition and after moving the device.

11. The method of claim 2 or any preceding claim when depending on claim 2, and the fluid having a rate of evaporation sufficiently slow to enable a delay before starting the directing step.

12. A biosensor device having a substrate (10), magnetic or magnetisable particles (60) deposited at a free area of the substrate (10) for binding to a target being detected, the device having a magnetic field sensor (40) for detecting the magnetic or magnetisable particles (60) after binding, and having a built in magnetic field generation means (260, 270) arranged to direct the magnetic or magnetisable particles (60) to the free area on the device away from the magnetic field sensor (40), to be available for later use in binding and detecting.

13. The device of claim 12 being an integrated micro-fluidic device having fluid handling elements and control circuitry (30) on the same substrate (10) as the magnetic field sensor (40).

14. The device of claim 12 or 13, the magnetic field sensor (40) being formed in an integrated circuit, (30) and the built in magnetic field generation means comprising a wire (260, 270) integrated in the same integrated circuit (30) of the device.
